# EUROPEAN PATENT APPLICATION

(11) **EP 1 834 653 A2**
(43) Date of publication of application: **19.09.2007**
(21) Application number: 06024465.4
(22) Date of filing: 08.02.2005
(51) Int. Cl.: A61L 9/12, B60H 3/00, A01M 1/20

(54) **Wick-based delivery system incorporating a capillary member**

(30) Priority: 13.02.2004 US 777079
(62) Divisional of application: 05713120.3
(71) Applicant: S.C. Johnson & Son, Inc., Racine, WI 53403 (US)
(72) Inventor: Crapser, James R., Racine WI 53406 (US); Houser, David J., Racine WI 53403 (US)
(74) Representative: Ruschke, Hans Edvard

(57) **Abstract**

An evaporative device including a container (1) for holding a liquid, the container (1) having an opening; a porous wick (3a) extending through the opening such that a portion of the wick (3a) contacts the liquid held within the container (1) and a portion of the wick (3a) is exposed to the ambient environment, wherein the wick (3a) transfers the liquid from the container (1) to the ambient air, and a capillary insert member (8) having a surface in communication with a bore in the end of the wick (3a). The surface of the insert member has one or more exposed capillary pathways (9) along which liquid, transferred by the wick (3a) from the container, is drawn by capillary action for dispersion to the ambient air. The axial portion of the insert in the bore can be adjusted automatically in response to temperature thus achieving a constant evaporation rate despite temperature variations.

## Description

### BACKGROUND OF THE INVENTION

### 1. Field of the Invention

Our invention relates to a device for transporting liquids, such as insect repellants, fragrances, or insecticides, from a reservoir to a surface exposed to the ambient air.

### 2. Description of the Related Art

Devices that release vapors into the ambient air are well-known in the art. Generally, the purpose of these devices is to deodorize, provide fragrance to, and/or disinfect the ambient air, or to distribute toxins into the air to kill or repel unwanted pests, such as mosquitoes.

To achieve the goal of dispersing active particles into the air, a number of methods has been employed. For example, aerosol containers have been used to atomize particles into the air upon the activation of a trigger by a user.

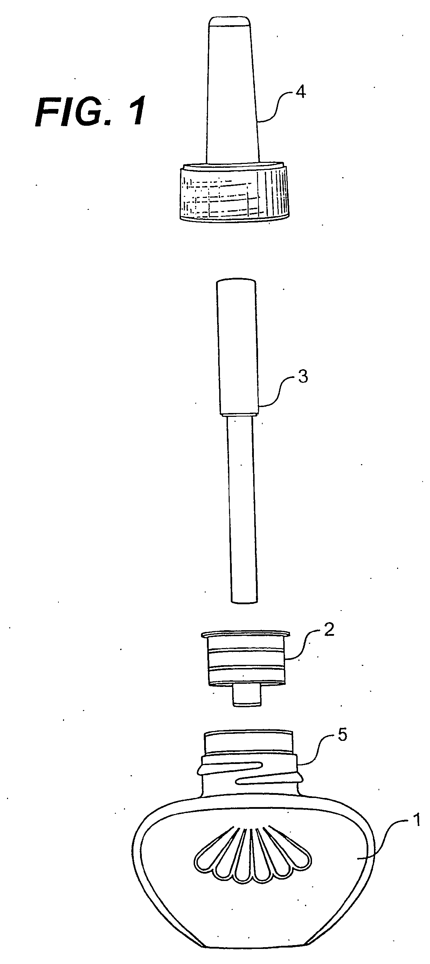

Also, other methods utilize the evaporative properties of liquids, or other vaporizable materials, to cause vapors with desired properties to be distributed into the ambient air. One such known evaporative technique, illustrated in Figure 1, utilizes a wick to deliver a vaporizable liquid from a reservoir to a surface exposed to the ambient air. An example of such a product is GLADE® PLUGINS® Scented Oil, by S.C. Johnson & Son, Inc. (Racine, WI).

As shown in Figure 1, the reservoir from which the liquid is transported is a bottle 1 having a conventional shape. The bottle 1 contains a vaporizable liquid. (The level of the liquid is not shown in the bottle 1.) A wick 3 is preferably shaped to fit snugly into a neck 5 of the bottle 1. It is also preferable to use a neck closure 2 to hold the wick 3 in place and to prevent leakage around the neck 5 of the bottle 1. The fit between the neck closure 2 and the bottle 1 should be sufficient to prevent leakage of the liquid from the bottle 1. Likewise, the fit between the neck closure 2 and the wick 3 should be sufficient to prevent leakage.

When assembled, the wick 3 is arranged such that a portion thereof is in contact with the liquid and another portion thereof is exposed to the ambient air. Arranged as such, the wick 3 transports the liquid to the surface of the wick by a principle called capillary action. In particular, the wick 3 material contains numerous small, internal, interconnecting pores. When liquid contacts those pores, it is elevated by principles of surface tension due to attractive forces, causing the liquid to be drawn into adjacent pores. As this process continues, the liquid migrates through the porous material. As the liquid is drawn from the bottle 1, it is transported up the porous wick 3 and eventually reaches a surface of the portion of the wick 3 exposed to the ambient air. As the liquid reaches this exposed surface, the liquid evaporates and disperses into the air.

While, as just described, it is conventionally known to expose a surface of the wick to the air, it is also known to provide multiple porous members, in fluid communication with each other, with a surface of one porous member being the exposed surface and another porous member contacting the fluid. For example, as shown in U.S. Patent No. 4,413,779, a glass container contains a fluid into which two rigid porous nylon wicks extend. The wicks also contact a rigid plastic porous element. In use, the wicks transport the fluid from the glass container to the porous element, which releases the fluid to the ambient air.

Such wick-based evaporative methods have become relatively commonplace and are effective at dispersing an evaporative liquid to the ambient air. However, they do have drawbacks. In fact, a prevalent problem associated with these methods is their inability to provide a linear release of the liquid to be emanated to ambient air. For example, while a given amount of liquid is emanated during the first day of use of a wick-based air freshener, the amount emanated decreases continually through successive days and weeks.

As an attempt to overcome the problems associated with conventional evaporative devices, some devices provide heat to the wick surface, some utilize electric fans, and some a combination of the two. Generally, the combined apparatus is designed to be plugged into an electrical outlet, to provide power for the electrically operated features. The heater raises ambeient temperature adjacent to the wick, thereby aiding release of the liquid, while the fan blows a stream of air across the wick. In theory, this fan increases the rate of emanation of the liquid from the wick. While in such systems the evaporative device used in conjunction with the heating device and/or the fan can usually be replaced by a refill, thereby allowing the electrically operated components to be reused, the heater device, fan, and necessary electronic components increase the overall price of the evaporative system.

A further method of dispensing vapors into the air is illustrated in U.S. Patent No. 4,913,350. According to this disclosure, an external capillary member is partially in contact with a liquid fragrance contained in a reservoir and partially in contact with the ambient air. The external capillary member has one or more external capillary cavities formed in the outer surface of the external capillary members. Like the porous cavities in conventional wicks, the capillary cavities draw the liquid to be emanated from the reservoir to the portion of the capillary member exposed to the ambient air. Once exposed, the liquid is released into the ambient air.

The use of the capillary channels is seen to be advantageous because, theoretically, the fragrance is delivered to the ambient air at a more constant rate, thereby overcoming the above-discussed problem associated with the conventional porous wick. In practice, however, open capillary release systems also have drawbacks. Most noticeably, the capillary member has proven to be inferior, and even ineffective, at removing many liquid formulations from a reservoir (i.e., depending on viscosity, surface tension, etc.).

As such, there is a need in the art for a cost-effective evaporative method of releasing a liquid into the ambient air that reliably releases the liquid at a constant rate.

### SUMMARY OF THE INVENTION

An object of our invention is to provide a cost-effective, wick-based evaporative device that remedies those problems discussed above.

In one aspect of our invention, an evaporative device includes a container, a porous wick, and a capillary member. The container holds a liquid and has an opening. The porous wick extends through the opening in the container such that a portion of the wick contacts the liquid held within the container and a portion of the wick is exposed to the ambient air. The wick transfers the liquid from the container. The capillary member has a surface in communication with a portion of the wick. One or more capillary pathways are disposed on the surface of the capillary member along which liquid, transferred by the wick from the container, is drawn by capillary action for dispersion to the ambient air.

According to another aspect of our invention, an evaporative device includes a container, a porous wick, and a capillary plate. The container holds a liquid and has an opening. The porous wick extends through the opening such that a portion of the wick contacts the liquid held within the container and a portion of the wick extends outside of the container. The wick transfers the liquid from the container. The capillary plate has a surface in communication with a portion of the wick. The surface has one or more capillary pathways along which liquid, transferred by the wick from the container, is drawn by capillary action for dispersion to the ambient environment.

According to yet another aspect of our invention, an evaporative device includes a container, a porous wick, and a capillary insert. The container holds a liquid and has an opening. The porous wick has an aperture extending axially therein and the wick extends through the opening of the container such that a portion of the wick contacts the liquid held within the container and a portion of the wick is exposed to the ambient air. The wick transfers liquid from the container. The capillary insert is insertable into the aperture in the wick, such that a surface of the capillary insert is in communication with the wick. The surface has a plurality of capillary pathways along which liquid transferred by the wick from the container is drawn by capillary action for dispersion to the ambient environment.

According to a further aspect of our invention; an evaporative system includes a container, a porous wick, a capillary insert, a housing, and a heat sensitive member. The container holds a liquid and has an opening. The porous wick has an aperture extending axially therein forming an inner surface of the wick that is exposed to the ambient environment, and the wick extends through the opening of the container such that a portion of the wick contacts the liquid held within the container and a portion of the wick is exposed to the ambient air. The wick transfers liquid from the container to the inner surface of the wick. The capillary insert is insertable into the aperture in the wick, thereby forming a slidable engagement between the inner surface of the wick and a surface of the capillary insert. The surface has one or more capillary pathways along which liquid, transferred by the wick from the container, is drawn by capillary action for dispersion to the ambient air. The housing contains at least a portion of one or more of the container, the porous wick, and the capillary insert. The heat sensitive member is in communication with both the housing and the capillary insert and varies in length based on ambient temperature. When the heat sensitive member varies in length, the change in length causes displacement of the capillary insert within the aperature of the work.

A better understanding of these and other features and advantages of our invention may be had by reference to the drawings and to the accompanying description, in which preferred embodiments of the invention are illustrated and described.

### BRIEF DESCRIPTION OF THE DRAWINGS

Figure 1 is an elevational, exploded view of a conventional wick-based delivery system.

Figure 2 is a perspective view of a wick-based delivery system according to an embodiment of our invention.

Figures 3A and 3B are perspective views of a wick-based delivery system according to further embodiments of our invention.

Figure 4 is a perspective view of a wick-based delivery system according to another embodiment of our invention.

Figure 5 is an elevational view of a wick-based delivery system according to a still further embodiment of our invention.

Figure 6 is a top view illustrating the relationship of the porous wick and the capillary member of Figure 5.

Figure 7 is an elevational, partial cut-away view of a wick-based delivery system according to yet another embodiment of our invention.

Throughout the figures, like or corresponding reference numerals have been used for like or corresponding parts.

### DETAILED DESCRIPTION OF THE PREFERRED EMBODIMENTS

Our invention relates generally to a wick-based delivery system for transporting a liquid from a reservoir to a surface that is exposed to the ambient air. Specifically, our invention relates to an improvement for a conventional wick-based system, like that shown in Figure 1.

As discussed above, Figure 1 illustrates a conventional wick-based delivery system in its simplest form. Our invention may be configured to include many of the base features of a conventional device. In particular, the system includes a bottle 1 containing a liquid (not shown in Figure 1) and a wick 3. Preferably, a neck closure 2 holds the wick 3 snugly in place within the neck 5 of the bottle 1. The wick 3 is preferably fixed in the bottle 1.

In addition, the neck 5 of the bottle 1 can be shaped so that a cap 4 can be securely fastened over the wick 3 and the neck closure 2. For example, an outer surface of the neck 5 of the bottle 1 may be threaded so that the cap 4 can be screwed onto the bottle 1 when the device is not in use.

The bottle 1 and the neck closure 2 can be made of any suitable material that is leakproof. Of course, the size of the opening in the bottle 1 and the size of the neck closure 2 are dependent upon each other and upon the size of the wick 3 that is to be used with the device.

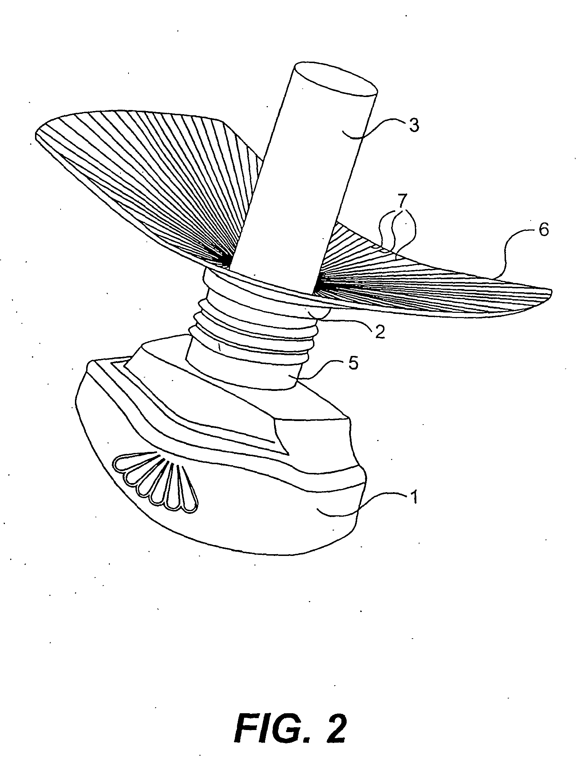

Turning now to Figure 2, a preferred embodiment of our invention will be described. As can be seen in Figure 2, the evaporative device of Figure 1 is assembled, and attached thereto is a capillary member, preferably, a capillary plate 6. The capillary plate 6 is preferably nonporous and includes one or more capillary channels 7 thereon. At least a portion of the capillary channels 7 is in substantial fluid communication with the wick 3. The capillary channels 7 are exposed to the ambient environment and are, individually, continuous from the position at which they are in intimate communication with the wick 3 to peripheral portions of the capillary plate 6. Other variations of channel-like capillaries may be used based on design requirements. In particular, in other embodiments, portions of the capillary channels 7 do not need to be exposed to the ambient environment.

As illustrated, the capillary plate 6 is substantially wing-shaped, although we contemplate many suitable shapes and sizes. Also, according to this embodiment, the capillary plate 6 is preferably approximately .750 (1.905 cm) inches wide by 1.50 inches (3.81 cm) long, although such is merely exemplary. According to design preferences, the capillary plate 6 should be sized to effectuate an optimal release of fragrance. This sizing will likely be based on at least, the properties of the liquid used, the emanation rate of the wick 3, and various emanation preferences.

As shown in Figure 2, the capillary channels 7 are preferably V-shaped in cross section. Also, the V-shaped capillary channels 7 are preferably 0.007 inches (0.1778 mm) wide at the surface of the capillary plate 6, 0.002 inches (0.0508 mm) wide at the base of the channel, and 0.017 inches (0.438 mm) deep. Notwithstanding, other shapes and sizes may be equally effective at transporting the liquid via capillary action from the wick 3. Generally, any shape and size that allows the liquid contained within the bottle 1 to be transported by capillary action will suffice. Further, as will be apparent to one of ordinary skill, the number or length of the capillary channels 7 will have a direct effect on the amount of liquid emanated from the capillary member. Specifically, as the number or length of the capillary channels 7 increases, the greater the amount of liquid emanated. Likewise, as the number or length of the capillary channels 7 decreases, the lesser the amount of liquid emanated. While the makeup of the capillary channels 7 will in this manner influence an amount of emanation, one of skill in the art will of course understand that the physical properties of the liquid will also dictate the emanation amount.

Also, although not required, we have found that when a V-shaped cross section is used for the capillary channels 7 on the capillary plate 6, it is preferable for the point, or apex, of the V-shape to be in contact with the wick 3. Such an arrangement provides an effective way of drawing, via capillary action, evaporative liquid from the wick 3. Such is not required, however.
Any contact point on a capillary channel 7 that fosters capillary action will promote the accumulation of liquid within that capillary channel 7 and will thus achieve the desired emanation.

Further, while the capillary channels 7 are shown on the top (i.e., the side furthest from the bottle 1) of the capillary plate 6, such is not required. In fact, we contemplate that the capillary channels 7 may be formed on the underside (i.e., the side closest to the bottle 1) of the capillary plate 6. By placing the capillary channels 7 on the underside, for example, dust and other airborne impediments are less likely to collect in the capillary channels 7. In other embodiments, the capillary member may reside in other orientations relative to the porous wick. By way of illustration, the capillary member may be disposed such that the capillary channels 7 are positioned lengthwise against the wick 3.

Moreover, although in this preferred embodiment, the capillary channels 7 are used, any surface having exposed capillary pathways along which a liquid can be drawn may be used as the capillary member. Further, the capillary pathways may be continuous from the wick 3 to the periphery of the capillary plate 6, like the capillary channels 7 of Figure 2, or otherwise arranged to provide flow of liquid. In preferred embodiments, the capillary plate 6 is non-porous, such that liquid can flow along the capillary pathways, on a surface thereof, but not through the capillary plate 6, as would happen in a conventional wicking substrate.

In operation, the wick 3 is constructed such that the liquid contained within the bottle 1 is drawn through the porous wick 3 via capillary action. Then, as the liquid reaches an outer surface of the wick 3 exposed to the ambient air, the liquid evaporates, thereby releasing, for example, a liquid fragrance to the ambient air. Unlike conventional devices, however, as the liquid reaches the outer surface of the wick 3, the capillary channels 7 of the capillary plate 6 draw liquid, also by capillary action, from the wick 3. The liquid drawn to capillary plate 6 is also drawn along capillary channels 7.

As should thus be apparent, according to the embodiment shown in Figure 2, the liquid is introduced to the ambient air via both the outer surface of the wick 3 and the capillary plate 6. As a result, the amount of liquid released to the atmosphere is increased. We have found that the addition of the capillary channels 7 causes the release of the liquid to the ambient air at a more constant rate than can be achieved through the use of only a wick 3.
Essentially, this configuration allows for an increased, substantially constant emanation rate.

The capillary plate 6 of Figure 2 may also be detachably secured to the wick 3. For example, the capillary plate 6 may be manufactured so that an aperture through the capillary plate 6 allows the capillary plate 6 to be slid over the wick 3. The bottom of the capillary plate 6 may come to rest on the neck closure 2 of the bottle 1 or on the neck 5 of the bottle 1. Alternatively, the outer perimeter of the wick 3 and the aperture through the capillary plate 6 may be formed so as to create an interference fit such that the capillary plate 6 comes to rest on an elevation of the protruding wick 3. In either design, liquid is drawn from the wick 3 to the capillary plate 6. When this is achieved, the capillary channels 7, because of their communication with the wick 3, further disperse the liquid drawn by the wick 3.

By making the capillary plate 6 detachably securable, the capillary plate 6 can preferably be reused with a replacement evaporative device. Specifically, when all of the liquid contained within the bottle 1 of the embodiment shown in Figure 2 is dispersed, the capillary plate 6 can be detached and reattached to a replacement evaporative device. Such a replacement generally includes those parts shown in Figure 1, although it only need include a liquid, a container for the liquid, and a wick.

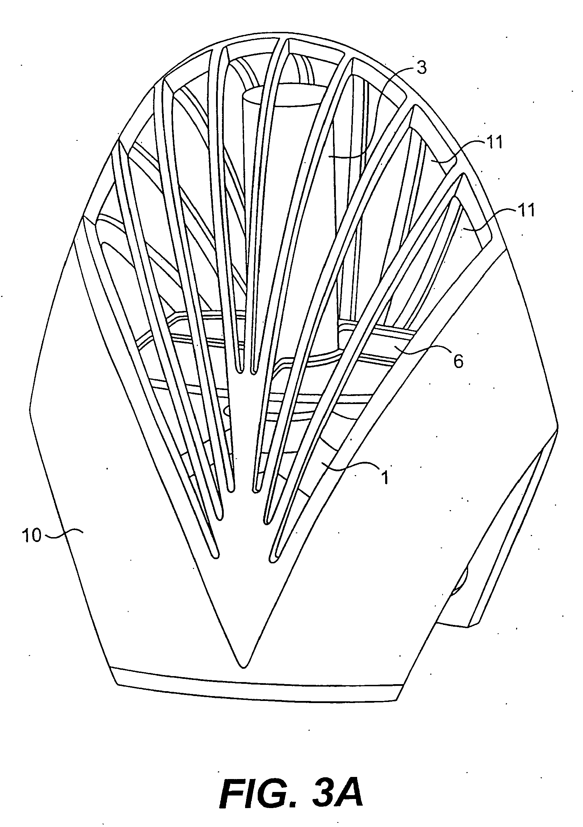

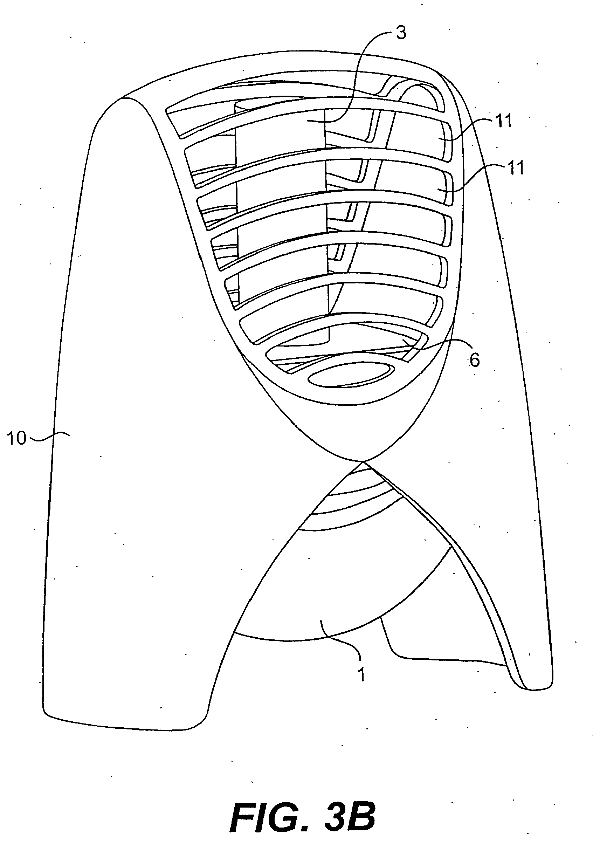

The embodiment of Figure 2 shows all of the components just discussed as an integral unit, designed to stand alone as, for example, an air freshener. We also envision that the unit could be designed to removably attach to a housing 10. Illustrative examples of this embodiment are depicted in Figures 3A and 3B. As shown in these figures, a housing 10 having one or more vents 11 therethrough is used to contain the evaporative device of Figure 2. Preferably, the housing 10 and the bottle 1 are manufactured such that an outer surface of the bottle 1 can be engaged with an inner surface of the housing 10, although any detachable securement will suffice. By making the housing 10a part of the device, the overall aesthetics of the device are improved and the surfaces of the wick 3 and the capillary plate 6 are not exposed to external forces that may result in, for example, damage to the wick 3. The vents 11 allow liquid vapors emanated from the wick 3 and/or the capillary plate 6 to freely pass into the ambient air.

An advantage of making the housing 10 and the emanation device detachable from each other is that the housing 10 may be reused. Generally, once the liquid contained within the bottle has evaporated completely, the entire device, i.e., the bottle 1, the wick 3, and anything connected thereto, are disposed of, and a replacement is purchased. However, since the housing 10 is a separate unit from which the device can be removed, a replacement device can be purchased and inserted into the housing 10. While we contemplate that the capillary plate 6 may be an integral portion of the evaporative device and thereby sold as part of the replacement device, we also contemplate the capillary plate 6 being fixed to the housing 10. As such, when all of the liquid within the bottle is used, a replacement for detachable attachment to the housing 10 does not include the capillary plate 6, thereby giving the user the benefits associated with the capillary plate 6, but without having to purchase it as part of a replacement.

As shown in Figures 3A and 3B, for example, the housing 10 contains the capillary plate 6 such that, when a replaceable wick-based evaporative system is contained within the housing 10, the wick 3 and the capillary plate 6 are in contact with each other. However, when the liquid supply is exhausted from the device, only the bottle 1, wick 3, and neck closure 2 (if used) are disposed of. A replacement contains only these components, as well as a fresh supply of evaporative liquid. As discussed above, the mating of the wick 3 and the capillary plate 6 may be one of several possible so that the wick 3 is in fluid contact with the capillary channels 7 of the capillary plate 6 when the replacement is inserted.

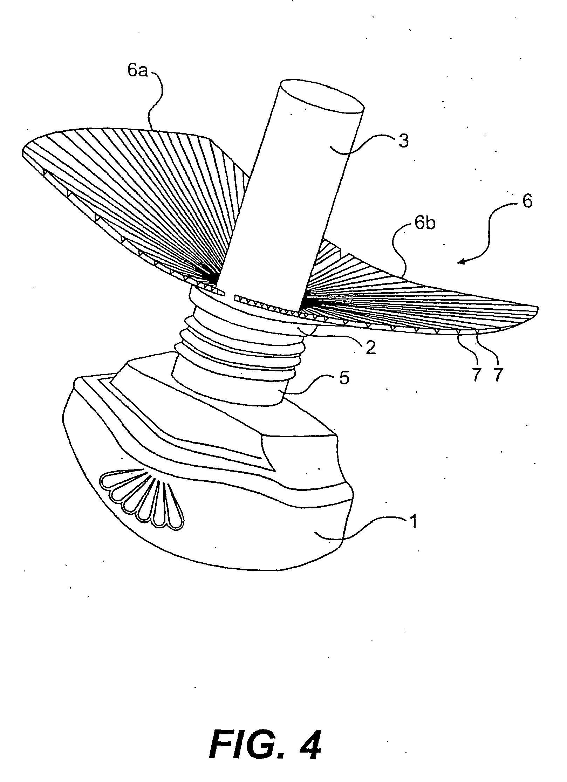

Illustrating another preferred embodiment of our invention, Figure 4 shows a device similar to that of Figure 2, with the exception that the capillary plate 6 consists of two portions, i.e., capillary partial-plates 6a, 6b. While the operation of the device of Figure 4 is substantially the same to that of the device of Figure 2, using the capillary partial-plates 6a, 6b allows one to regulate the amount of liquid released to the ambient air. In particular, when both of the capillary partial-plates 6a, 6b are in communication with the wick 3, the device acts as the embodiment illustrated in Figure 2. However, when one of the capillary partial-plates 6a, 6b is no longer in communication with the wick 3, less liquid is emanated to the ambient air. Emanation decreases because the liquid transferred from the bottle 1 by the wick 3 will only transfer through those capillary channels 7 that remain in contact with the wick 3. When neither of the capillary partial-plates 6a, 6b are in contact with the wick 3, the device acts as the conventional device illustrated in Figure 1, discussed above.

In order to impede the capillary channels 7 of one or both of the capillary partial plates 6a, 6b from contacting the wick 3, the capillary partial plates 6a, 6b may either be entirely removable from the device, or they may be actuatable in a direction away from the wick 3. Thus, in this embodiment, the capillary partial plates 6a, 6b function to transfer the liquid from the wick 3 for release to the ambient air only when in contact with the wick 3. Moreover, because the capillary partial plates 6a, 6b can be actuated away from the wick 3, the wick 3 is thus allowed to be inserted and withdrawn for purposes of replacement when no liquid remains. Also, when the capillary partial plates 6a, 6b are removed from communication with the wick 3, less liquid is emanated. As such, the useful life of the refill may be extended.

Furthermore, while the embodiment of Figure 4 shows only two capillary partial- plates 6a, 6b, we anticipate that more capillary partial-plates 6a, 6b could be utilized. As the underlying purpose of the capillary partial-plates 6a, 6b is to provide for an adjustable emanation device, it should be understood that the more capillary partial-plates 6a, 6b that are utilized, the greater the capability for adjustment.

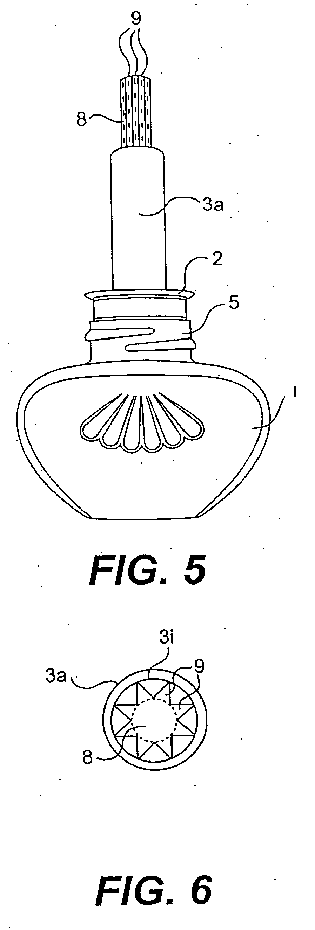

Turning now to another embodiment of our invention, Figure 5 shows an emanation device including a bottle 1 with a neck 5, a neck closure 2, a porous wick 3a, and a capillary member formed as a capillary insert 8, with one or more capillary channels 9 formed thereon. The function and construction of the bottle 1, the neck 5, the neck closure 2, and the wick 3a is similar to that of the embodiments discussed previously. In this embodiment, however, the wick 3a has an aperture formed in an axial direction therein, creating an inner surface 3i of the wick 3a, as seen in Figure 6. While the aperture may extend throughout the entire length of the wick 3a, thereby rendering the wick 3a hollow, the aperture may only extend partially along the length of the wick 3a, thereby forming a bore in the wick 3a. Regardless of the length of the aperture, it should be understood that the inner surface 3i of the wick 3a created as a result of the aperture is exposed to the ambient environment, and, as such, liquid transferred by the wick 3a from the bottle 1 is emanated therefrom.

The capillary insert 8 is designed for insertion into the aperture of the wick 3a and is preferably nonporous. When inserted, the capillary channels 9 of the capillary insert 8 are in communication with the inner surface 3i of the wick 3a. As would be appreciated, the presence of capillary insert 8 reduces (or prevents) the exposure of inner surface 3i to the ambient environment. However, as a result of this communication, the capillary channels 9 of the capillary insert 8 transfer liquid emanated from the inner surface 3i of the wick 3a, via capillary action, along their respective lengths.

Preferably, insertion of the capillary insert 8 into the aperture formed in the wick 3 a results in a slidable engagement between the capillary insert 8 and the inner surface 3i of the wick 3a. When the capillary insert 8 is slidable within the wick 3a, the amount of surface area of the capillary channels 9 that is exposed to the ambient air can be controlled. Specifically, when the capillary insert 8 is moved within the wick 3a closer to the bottle 1, less of the capillary insert 8 is exposed to the ambient air, above the wick 3a. As a result, relatively less liquid is emanated to the ambient air. Conversely, when the capillary insert 8 is moved within the wick 3a away from the bottle 1, more of the capillary insert 8 is exposed to the ambient air, above the wick 3a. As a result, relatively more liquid is emanated.

With this configuration, our invention allows a user to achieve a preferred amount of emanation from the wick 3a by performing a simple adjustment.

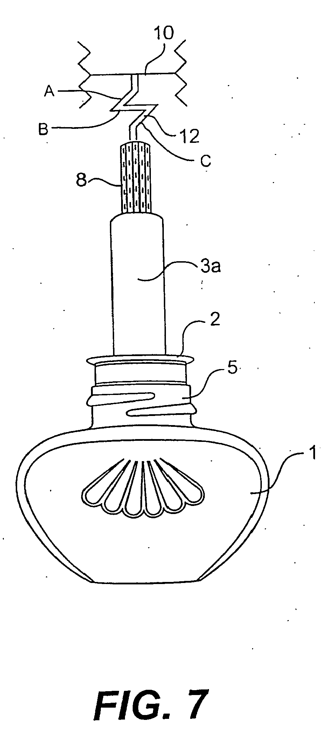

Figure 7 is a partial cut-away view illustrating still another embodiment of our invention. In Figure 7, the wick 3a and the capillary insert 8 are identical to those of the embodiments of Figure 5 and Figure 6. However, the embodiment of Figure 7 provides a self-adjusting feature. More specifically, the embodiment of Figure 7 is capable of automatically adjusting the position of the capillary insert 8 within the wick 3a based on a change in temperature.

As illustrated in Figure 7, the evaporative system is enclosed in a housing 10. The housing 10 may resemble those of Figures 3A and 3B, although it may be of myriad shapes and sizes. A heat sensitive member 12 is connected to both the housing 10 and the capillary insert 8. The heat sensitive member 12 is preferably substantially S-shaped and is sensitive to heat. The heat sensitive member 12 is preferably made of bimetal.

In operation, as the temperature of the heat sensitive member 12 decreases (as controlled by the ambient temperature), an overall vertical length of the heat sensitive member 12 shortens, as the three central portions A, B, and C of the heat sensitive member 12 each becomes more horizontally disposed. Conversely, as the temperature of the heat sensitive member 12 increases, the heat sensitive member 12 lengthens in a vertical direction, with the portions A, B, and C of the heat sensitive member 12 becoming more vertically disposed. As such, when attached to the housing 10 and the capillary insert 9, as shown in Figure 7, the shortening of the heat sensitive member 12 in cooler temperatures displaces the capillary insert 8 in a direction away from the bottle 1, and, therefore, more of the capillary insert 8 is exposed to the ambient air. Conversely, the lengthening of the heat sensitive member 12 in warmer temperatures displaces the capillary insert 8 in a direction toward the bottle 1, and, therefore, less of the capillary insert 8 is exposed to the ambient air.

This adjustability is advantageous because the temperature of the ambient air directly influences the rate of emanation of a liquid from a wick-based evaporative system. In particular, the warmer the ambient air, the quicker the rate of evaporation of the liquid, and, conversely, the cooler the ambient air, the slower the rate of evaporation of the liquid.

While the heat sensitive member 12 is shown in Figure 7 as being substantially S-shaped, we contemplate that the heat sensitive member 12 may be of many shapes, configurations, and/or materials. Furthermore, the embodiment shown in Figure 7 may be constructed such that, similar to that discussed with regard to the embodiment of Figures 3A and 3B, the housing 10 and the emanation device are detachably attachable with respect to each other. Ideally, the bottle 1, the wick 3a, and the neck closure 2 (if used) comprise a replacement that can be discarded when no liquid remains in the bottle 1. Accordingly, the housing 10, the heat sensitive member 12, and the capillary insert 8 are reused.

In addition, we have found that the embodiment of Figure 7 is particularly well suited for use in automobiles, in which temperature fluctuation is common. In a conventional device, an insufficient amount of liquid may be dispersed at cooler temperatures, while too much liquid may be dispersed at relatively warmer temperatures. Accordingly, the present embodiment is particularly well suited for this type of application, and the housing 10 may be specifically formed for mounting our evaporative system in an automobile. For example, the housing 10 may be detachably secured to a vent found in the automobile.

According to each of the embodiments discussed herein, liquid drawn from the bottle 1 by the wick 3 or 3a is dispersed to the ambient air via both the wick 3 or 3a and the capillary member. However, we also envision embodiments in which the emanation from the wick 3 or 3a to the ambient air is minimized. For example, the wick 3 or 3a exposed to the ambient air and not in contact with the capillary member may be encased by a cover. Alternatively, the wick 3 or 3a may be shortened such that only a minimal or no portion of the wick 3 or 3a extends outside of the bottle 1.

In each of the embodiments discussed above, the wick is preferably made of micro-porous plastic the capillary insert is made of polypropylene or PET by injection molding, and the liquid to be dispersed is fragrance oil. Our invention is not limited to these preferences, however. Other known materials may be substituted, as desired.

The embodiments discussed above are representative of preferred embodiments of the present invention and are provided for illustrative purposes only. They are not intended to limit the scope of the invention. Although specific structures, dimensions, components, etc., have been shown and described, such are not limiting. Modifications and variations are contemplated within the scope of our invention, which is intended to be limited only by the scope of the accompanying claims.
Further embodiments are set out below.
An evaporative device comprising: a container for holding a liquid, the container having an opening; a porous wick extending through the opening such that a portion of the wick contacts the liquid held within the container and a portion of the wick is exposed to the ambient environment, wherein the wick transfers the liquid from the container; and a capillary member having a surface in communication with a portion of the wick, wherein one or more capillary pathways are disposed along the surface of the capillary member along which liquid, transferred by the wick from the container, is drawn by capillary action for dispersion to the ambient air.
The capillary member may be a capillary plate having one or more capillary channels, and wherein a portion of the capillary channels may be in communication with a portion of the wick such that the capillary channels transfer liquid from the wick for dispersion to the ambient environment. The capillary plate may be substantially wing shaped.
An evaporative device comprising: a container for holding a liquid, the container haveing an opening; a porous wick extending through the opening such that a portion of the wick contacts the liquid held within the container and a portion of the wick extends outside of the container such that the wick transfers the liquid from the container; and a capillary plate having a surface in communication with a portion of the wick, wherein the surface has one or more capillary pathways along which liquid, transferred by the wick from the container, is drawn by capillary action for dispersion to the ambient environment.
The capillary plate may be nonporous. The capillary plate may be substantially wing shaped.
The exposed capillary pathways may be substantially continuous along their lengths. The exposed capillary pathways may comprise one or more capillary channels and a portion of the capillary channels may be in communication with a portion of the wick extending outside the container. The capillary channels may be substantially V-shaped in cross section. The capillary plate may be detachably secured to one or both of the wick and the container. The said surface may be one of a top and a bottom of the capillary plate. The evaporative device may further comprise a cover that encases a portion of the portion of the wick extending outside of the container. The evaporative device may have plural capillary plates, each having one or more capillary pathways, and the capillary pathways may be in communication with the portion of the wick extending outside of the container. The plural capillary plates may be movable such that the capillary pathways of each are removable from communication with the portion of the wick extending outside of the container. The plural capillary plates may be actuatable in a direction away from the wick to separate the capillary pathways thereof from communication with the portion of the wick exposed to the ambient air. The capillary pathways may be exposed on the surface of the capillary plate. The capillary plate may be composed of polyethylene.
An evaporative system comprising: an evaporative device as described above; and a housing for containing at least a portion of the evaporative device.
The evaporative device may be detachably attached to the housing.
The capillary plate may be fixed to the housing, and the container and the wick may be detachably attachable to the housing and the capillary plate.

### INDUSTRIAL APPLICABILITY

Our invention provides a device useful as a means to transport a liquid from a container to a surface that is exposed to the ambient air. The surface may be either a surface of a porous wick, a capillary member having one or more capillary channels, or a combination of both a surface of a porous wick and a capillary member having one or more capillary channels. This device can preferably be used, for example, to dispense fragrances, insecticides, and any other vaporizable materials into the ambient air.

## Claims

1. An evaporative device comprising:
a container (1) for holding a liquid, the container having an opening:
a porous wick (3a) extending through the opening such that a portion of the wick contacts the liquid held within the container and a portion of the wick is exposed to the ambient environment, wherein the wick transfers the liquid from the container; and
a capillary member (8) having a surface in communication with a portion of the wick, wherein one or more capillary pathways are disposed along the surface of the capillary member along which the liquid, transferred by the wick from the container, is drawn by capillary action for dispersion to the ambient air;
**characterized in that** the wick (3a) has an axial aperture and the capillary member comprises an insert (8) designed to fit in the aperture, said capillary pathways comprising capillary channels (9) formed on the insert (8) such as to be in communication with an inner surface (3i) of the aperture of the wick (3a), to transfer liquid from said inner surface (3i) to said channels (9).

2. A device according to claim 1 wherein the insert (8) is non-porous.

3. A device according to claim 1 or 2 wherein the insert is in slidable engagement with the inner surface (3i) of the wick (3a) so that the amount of surface area of the capillary channels that is exposed to the ambient air is controllable.

4. A device according to claim 3 further including a housing (10),
a container with a capillary member being enclosed by the housing (10); and
a heat sensitive member (12) connected both to the housing and the capillary insert (8) such that in warmer temperatures the heat sensitive member (12) displaces the insert (8) in a direction towards the bottle thereby reducing the amount of the insert (8) that is exposed to ambient air and in cooler temperature the heat sensitive member (12) displaces the insert in a direction away from the bottle thereby exposing more of the insert (8) to the ambient air.

5. A device according to claim 4 wherein the heat sensitive member (12) is substantially S-shaped, and preferably bi-metallic.

6. A device according to any preceding claim wherein a cover is provided encasing the exposed portion of the wick that is not in contact with the insert (8).

7. A device according to any preceding claim wherein the wick is made of micro-porous plastic and the insert is made of polypropylene or PET by injection molding.

8. An evaporative device comprising:
a container (1) for holding a liquid, the container having an opening:
a porous wick (3, 3a) extending through the opening such that a portion of the wick (3, 3a) contacts the liquid held within the container (1) and a portion of the wick (3, 3a) is exposed to the ambient environment, wherein the wick (3, 3a) transfers the liquid from the container (1); and
a capillary member (6, 8) having a surface in communication with a portion of the wick, wherein one or more capillary pathways (7, 9) are disposed along the surface of the capillary member (6, 8) along which the liquid, transferred by the wick from the container, is drawn by capillary action for dispersion to the ambient air;
**characterized in that** said capillary member (6, 8) comprises a plurality of plates (6a, 6b) each selectively actuable towards and away from wick, or an insert (8) slidably movable in an axial aperture in the end of the wick, to control the amount of evaporation of liquid from the capillary member.

9. An evaporative device system comprising:
a housing for supporting a bottle of liquid to be evaporated having a wick protruding from a neck of the bottle,
a capillary member (6, 8) attached to the housing and having a surface adapted to communicate with the wick, said surface having one or more capillary pathways for transferring liquid from the wick to be dispensed to the ambient air,
**characterized in that** an actuator is provided to move the capillary member or portions thereof thereby controlling the amount of liquid dispensed to the ambient air, wherein the capillary member is optionally an insert (8) suitable to be slidably receivable in an axial aperture in a wick (3a).

10. A refill for an evaporative device system comprising:
a container (1) for holding a liquid, the container having an opening; and
a porous wick (3a) extending through the opening such that a portion of the wick contacts the liquid held within the container and a portion of the wick is exposed to the ambient environment, wherein the wick transfers the liquid from the container; **characterized in that** the wick (3a) has an axial aperture adapted to receive an insert (8) designed to fit in the aperture, capillary channels (9) formed on the surface of the insert (8) in communication with an inner surface (3i) of the aperture to transfer liquid transferred by the wick from the container from said inner surface (3i) by capillary action to said channels (9) for dispersion to the ambient air.
